# EUROPÄISCHE PATENTANMELDUNG

(11) **EP 1 428 648 A2**
(43) Veröffentlichungstag der Anmeldung: **16.06.2004**
(21) Anmeldenummer: 03028080.4
(22) Anmeldetag: 09.12.2003
(51) Int. Cl.: B29C 70/08, A61F 5/02

(54) **Verfahren zum Herstellen einer orthopädischen Stütze**

(30) Priorität: 14.12.2002 DE 10258556
(71) Anmelder: Kriwat, Holger, 24103 Kiel (DE); Kriwat, Michael, 24103 Kiel (DE)
(72) Erfinder: Kriwat, Holger, 24103 Kiel (DE); Kriwat, Michael, 24103 Kiel (DE)
(74) Vertreter: Tönnies, Jan G.

(57) **Zusammenfassung**

Verfahren zum Herstellen einer orthopädischen Stütze mit Bereichen unterschiedlicher Festigkeit mit den folgenden Schritten: Auswählen eines der Form der Stütze entsprechenden Formwerkzeugs, inlegen eines Fasergewirks in die Zone des Formwerzeugs, die dem mit größerer Festigkeit auszubildenden Bereich der Stütze entspricht, und Einbringen eines aufschäumend aushärtenden Werkstoffs in den durch das Formwerkzeug gebildeten Raum unter Tränken des Fasergewirks.

## Beschreibung

Die Erfindung betrifft ein Verfahren zum Herstellen einer orthopädischen Stütze mit Bereichen unterschiedlicher Festigkeit.

Orthopädische Stützen finden zur Behandlung und Stützung bei unterschiedlichen Fehlbildungen des menschlichen Körpers Verwendung, beispielsweise als Schuheinlagen, Rückenschienen und Sprunggelenkstützen.

Es ist bei derartigen orthopädischen Stützen von Bedeutung, dass diese in bestimmten Bereichen polsternd oder dämpfend, also nachgiebig ausgebildet sind, in anderen Bereichen dagegen stützend oder führend, also fest ausgebildet sind, wobei die Stütze ein geringes Eigengewicht haben soll. Die heute verwendeten orthopädischen Stützen mit Bereichen unterschiedlicher Härte sind aus verschiedenen, nach Fertigung miteinander verklebten oder verschweißten Bereichen gefertigt. Diese Herstellung ist aufwendig, die fertige Stütze hat ein hohes Eigengewicht.

Aus der DE 38 04 321A1 ist ein Verfahren zum Herstellen einer orthopädischen Formstütze mit den Merkmalen des Oberbegriffs des Anspruchs 1 bekannt, bei dem ein Fasermaterial zunächst mit einem thermoplastisch verformbaren Kunststoff getränkt, sodann mit einer Kunststoffolie verpresst und sodann in einem Formwerkzeug verpresst wird. Ähnliche Verfahren offenbaren die DE 1491209 A und die DE 33 04 537 A1. Die Verwendung eines aufschäumenden Kunststoffs bei der Herstellung orthopädischer Stützen ist aus der DE 1790 894 U, der DE 1 037 920 B und der DE 87 00 682 U1 bekannt.

Der Erfindung liegt die Aufgabe zugrunde, ein Verfahren zur Herstellung einer orthopädischen Stütze zu schaffen, das es auf einfache Weise ermöglicht, Bereiche mit unterschiedlicher Festigkeit auszubilden.

Erfindungsgemäß wird diese Aufgabe gelöst durch die Merkmale des Anspruchs 1.

Die so hergestellte orthopädische Stütze mit Bereichen unterschiedlicher Festigkeit kann den jeweiligen Anforderungen entsprechend ausgebildet sein, das Fasergewirk kann an den jeweiligen Ort platziert sein, es kann den jeweiligen Anforderungen entsprechend dünner oder dicker sowie weicher oder härter ausgebildet sein.

Durch eine geeignete Wahl des ausschäumenden Kunststoffs kann die jeweils gewünschte Grundfestigkeit gewählt werden.

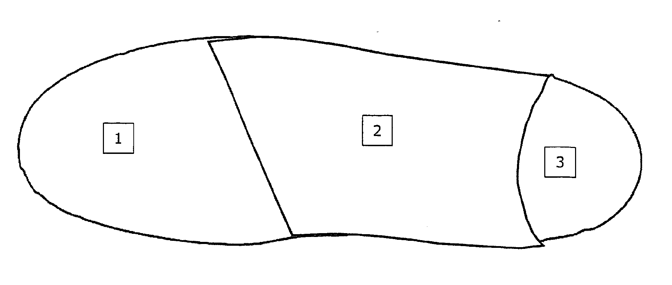

Die Erfindung wird im folgenden anhand einer Zeichnung erläutert. Dabei zeigt die einzige Figur eine Draufsicht auf eine orthopädische Schuheinlage als Beispiel einer erfinderisch ausgebildeten Stütze.

Die orthopädische Schuheinlage weist Bereiche 1, 2, 3 unterschiedlicher Festigkeit auf. Um dies zu bewirken wird ein der Form der Schuheinlage entsprechendes Formwerkzeugs ausgewählt, ein Fasergewirk in die Zone des Formwerkzeugs, die dem mit größerer Festigkeit auszubildenden Bereich 2 der Schuheinlage entspricht, eingelegt und ein aufschäumend aushärtenden Werkstoffs in den durch das Formwerkzeug gebildeten Raum unter Tränken oder Durchspritzen des Fasergewirks eingebracht.

## Patentansprüche

1. Verfahren zum Herstellen einer orthopädischen Stütze aus einem Kunststoff mit durch einen Faserwerkstoff verstärkten Bereichen unter Verwendung eines der Form der Stütze entsprechenden Formwerkzeugs, **gekennzeichnet durch**
Einlegen eines Fasergewirks in die Zone des Formwerkzeugs, die dem mit größerer Festigkeit auszubildenden Bereich der Stütze entspricht, und
Einbringen des aufschäumend aushärtenden Werkstoffs in den **durch** das Formwerkzeug gebildeten Raum unter Tränken des Fasergewirks.

2. Verfahren nach Anspruch 1, **dadurch gekennzeichnet, dass** die Stütze eine Schuheinlage ist.
